# EUROPEAN PATENT APPLICATION

(11) **EP 1 371 362 A1**
(43) Date of publication of application: **17.12.2003**
(21) Application number: 02077338.8
(22) Date of filing: 12.06.2002
(51) Int. Cl.: A61K 9/127, A61K 31/573

(54) **Composition for treatment of inflammatory disorders**

(71) Applicant: Universiteit Utrecht Holding B.V., 3584 CK Utrecht (NL)
(72) Inventor: Metselaar, Josbert Maarten, Dept. of Pharmaceutics, 3508 TB Utrecht (NL); Storm, Gerrit, Dept. of Pharmaceutics, 3508 TB Utrecht (NL); Wauben, Marca H. Michaela, Dept. of Immunology, 3508 TB Utrecht (NL)
(74) Representative: Prins, Adrianus Willem

(57) **Abstract**

A pharmaceutical composition for parenteral administration, comprising liposomes composed of non-charged vesicle-forming lipids, optionally including not more than 5 mole percent of charged vesicle-forming lipids, the liposomes having a selected mean particle diameter in the size range between about 40 - 200 nm and containing a water soluble corticosteroid for the site-specific treatment of inflammatory disorders, is provided.

## Description

The present invention relates to a pharmaceutical composition for parenteral and in particular intravenous administration, comprising liposomes composed of non-charged vesicle-forming lipids, optionally including not more than 5 mole percent of charged vesicle-forming lipids, the liposomes having a selected mean particle diameter in the size range between about 40 - 200 nm and containing a corticosteroid for the site-specific treatment of inflammatory disorders.

### BACKGROUND OF THE INVENTION

Liposomes, which belong to the group of colloidal carrier particles, are small vesicles consisting of one or more concentric lipid bilayers enclosing an aqueous space. Because of their structural versatility in terms of size, surface charge, lipid composition, bilayer fluidity and because of their ability to encapsulate almost every drug, their importance as drug delivery systems was readily appreciated. However, on intravenous injecting of liposomes, these are recognised as foreign particles by the Mononuclear Phagocyte System (MPS) and rapidly cleared from the circulation to organs rich in phagocytic cells, like liver, spleen and bone marrow. Several possibilities to reduce this effect have been identified, such as decreasing the particle size of the liposomes and changing the surface charge of the liposomes. Another development relates to surface modification of the liposomes by the introduction of specific hydrophilic polymeric components on the liposomal surface, which groups reduce protein adsorption on the particle surface. Consequently such liposomes are protected against recognition by cells of the MPS and have a prolonged residence time in the general circulation. A well-known example of modification of the liposomal surface is the incorporation during the preparation of liposomal compositions of a lipid derivative of the hydrophilic polymer polyethylene glycol (PEG). Usually this polymer is terminus-modified with a hydrophobic moiety, which is the residue of a phosphatidyl ethanolamine derivative or a long-chain fatty acid. Polyethylene glycol per se is a rather stable polymer, which is a repellant of protein adhesion and which is not subject to enzymatic or hydrolytic degradation under physiological conditions. Good results with respect to extending plasma half life and diminishing accumulation into the organs rich in phagocytic cells have been obtained following intravenous administration of liposomes, having a PEG-grafted surface, to various animal species and also to human beings (Storm G., Belliot S.O., Daemen T. and Lasic D.D.: Surface modification of nanoparticles to oppose uptake by the mononuclear phagocyte system in Adv. Drug Delivery Rev. 17, 31-48, (1995); Moghimi S.M., Hunter A.C. and Murray J.C.: Long-circulating and target-specific nanoparticles; theory to practice in Pharmacol. Rev. 53, 283-318, (2001); Boerman O.C., Dams E.T., Oyen W.J.G., Corstens F.H.M. and Storm G.: Radiopharmaceuticals for scintigraphic imaging of infection and inflammation in Inflamm. Res. 50, 55-64, (2001)). Marketing approvals for such liposomal preparations, containing doxorubicine, have been obtained.

Meanwhile several disadvantages of the use of the polymer polyethylene glycol in long-circulating liposomes have been encountered. The accumulation of PEG-grafted liposomes in macrophages and the skin is of some concern due to non-biodegradability. Loss of the long-circulation property (fast clearance) on injecting PEG-liposomes for a second time has been observed (Dams E.T., Laverman P., Oijen W.J., Storm G., Scherphof G.L., Van der Meer J.W., Corstens F.H. and Boerman O.C.: Accelerated blood clearance and altered biodistribution of repeated injections of sterically stabilized liposomes in J. Pharmacol. Exp. Ther. 292, 1071-1079, (2000)). Recent studies with PEG-liposomes in patients have shown that PEG-liposomes can induce acute side effects (facial flushing, tightness of the chest, shortness of breath, changes in blood pressure), which resolve immediately when the administration (infusion) of the PEG-liposome formulation is terminated. Recent data point to a role of complement activation in the induction of side effects (Szebeni J., Baranyi L., Savay S., Lutz H., Jelezarova E., Bunger R. and Alving C.R.: The role of complement activation in hypersensitivity to Pegylated liposomal doxorubicin (Doxil) in J. Liposome Res. 10, 467-481, (2000)). Until now the commercially available preparations based on PEG-liposomes are aqueous suspension preparations. It is well-known that the shelf life of liposomal aqueous suspension preparations in general and also of PEG-liposomes is rather limited. Several techniques how to remove the vehicle or continuous phase of such preparations are known, such as, spray-drying, diafiltration, rotational evaporation etc., and preferably freeze-drying. Recently a freeze-drying method, which improved the long term shelf life of PEG-liposomes, containing the technetium-chelator hydrazino nicotinamide, was proposed (Laverman P., van Bloois L., Boerman O.C., Oyen W.J.G., Corstens F.H.M. and Storm G.: Lyophilisation of Tc-99m-HYMC labelled PEG-liposomes in J. Liposome Res. 10(2&3), page 117-129 (2000)), but further investigations into the results and applicability of this technique to liposomal preparations are required.

Long-circulating small-sized liposomes, which contain non-charged or slightly negatively charged vesicle-forming lipids, such as PEG-liposomes, after intravenous administration can circulate for many hours in a volume not larger than the general circulation and therefore, in theory, are able to deliver relatively high portions of anti-inflammatory agents via extravasation at sites of enhanced vascular permeability common to inflamed regions. Such liposomes are of particular interest in the treatment of inflammatory diseases, e.g. rheumatoid arthritis, which is a chronic autoimmune disorder, causing joint inflammation and progressive cartilage destruction. Although several types of antirheumatic drugs are available for use, the treatment of severe, persistent synovitis and acute exacerbations may require the use of several intravenous injections containing high doses of glucocorticoids. Although systemic corticosteroids can suppress the symptoms of the disease, adverse effects limit their use. In addition to this, glucocorticoids suffer from unfavourable pharmacokinetic behaviour: short plasma half-life values and a large distribution volume require high and repeated administration in order to reach a therapeutically effective concentration of the drug at the desired site of action. Intra-articular injection of steroids into the affected joints is often used to increase the (local) efficacy of the glucocorticoids and diminish the systemic adverse effects, but this way of administration is less comfortable for the patients and not feasible when multiple small joints are affected. Also, a significant incidence of painless destruction of the joint may be associated with repeated intra-articular injections of glucocorticoids. According to EP-0662820-B preferred compounds for entrapment in PEG-containing liposomes are the steroidal anti-inflammatory compounds, such as prednisone, methylprednisolone, paramethazone, 11-fludrocortisol, triamcinolone, betamethasone and dexamethasone. The steroids listed belong to the group of steroids which are systemically administered. However, no examples of long-circulating liposomes containing these glucocorticoids were provided. The only example of a glucocorticoid-containing PEG-liposome, viz. no. 12, related to the preparation of beclomethasone dipropionate-containing PEG-liposomes. On preparing dexamethasone-containing PEG-liposomes according to the disclosure in EP-0662820 and on intravenous administration of the same in an in vivo experimental arthritis model, the present inventors noted that the beneficial effects, as taught in EP-0662820, could not be observed at all.

Since glucocorticoids often are the most effective drugs in the treatment of inflammatory disorders, there is a need to provide liposomal compositions which after parenteral administration can more efficiently deliver effective amounts of glucocorticoid at the inflamed region or tissue for enhanced and prolonged local activity, also after repeated administration.

### SUMMARY OF THE INVENTION

It is an object of the present invention to provide a pharmaceutical composition for parenteral administration, comprising liposomes composed of non-charged vesicle-forming lipids, optionally including not more than 5 mole percent of charged vesicle-forming lipids, the liposomes having a selected mean particle diameter in the size range between about 40-200 nm and containing a water soluble corticosteroid for the site-specific treatment of inflammatory disorders.

### LEGENDS TO THE FIGURES

Figure 1 is a graphical representation of the mean values for the calculated percentage injected dose in plasma samples versus time for PEG-liposomes versus non-polymer-coated DSPC-cholesterol liposomes of different particle sizes.
Figure 2 is a graphical representation of the plasma levels of free dexamethasone after injection of 10 mg/kg of three different liposomal dexamethasone phosphate preparations.
Figure 3 is a graphical representation of the paw inflammation score versus time before and after a single intravenous injection of saline and dexamethasone phosphate-containing liposomes.

### DETAILED DESCRIPTION OF THE INVENTION

It has now been found that by incorporating a water soluble form of a corticosteroid in long-circulating liposomes, composed of non-charged vesicle-forming lipids, optionally including not more than 5 mole percent of charged vesicle-forming lipids, the liposomes having a selected mean particle diameter in the size range between about 40 - 200 nm, an increased localisation and improved retention of the corticosteroid at inflamed tissue after one single intravenous injection of a pharmaceutical composition, comprising the said liposomes, can be reached and, as a consequence thereof, significant reversal of paw inflammation in the rat adjuvant arthritis model.

The long-circulation liposomes according to the present invention have a circulation half life of at least 6 hours, the circulation half life being defined as the time at which the second linear phase of the logarithmic liposomal clearance profile reaches 50% of its initial concentration, which is the extrapolated plasma concentration at t=0.

The particle size of the liposomes is preferably between 50 and 110 nm in diameter.

A water soluble corticosteroid in accordance with the present invention is a compound which is soluble 1 in ≤10 (w/v), as assessed in water or water buffered at physiologic values, e.g. at pH > 6.0, at a temperature between 15 and 25°C.

Water soluble corticosteroids which can be advantageously used in accordance with the present invention are alkali metal and ammonium salts prepared from corticosteroids, having a free hydroxyl group, and organic acids, such as (C₂ - C₁₂) aliphatic saturated and unsaturated dicarbonic acids, and inorganic acids, such as phosphoric acid and sulphuric acid. Also acid addition salts of corticosteroids can advantageously be encapsulated in the long-circulating liposomes. If more than one group in the corticosteroid molecule is available for salt formation, mono- as well as di-salts may be useful. As alkaline metal salts the potassium and sodium salts are preferred. Also other positively or negatively charged derivatives of corticosteroids can be used. Specific examples of water soluble corticosteroids are betamethasone sodium phosphate, desonide sodium phosphate, dexamethasone sodium phosphate, hydrocortisone sodium phosphate, hydrocortisone sodium succinate, cortisone sodium phosphate, cortisone sodium succinate, methylprednisolone disodium phosphate, methylprednisolone sodium succinate, methylprednisone disodium phosphate, methylprednisone sodium succinate, prednisolone sodium phosphate, prednisolone sodium succinate, prednisone sodium phosphate, prednisone sodium succinate, prednisolamate hydrochloride, triamcinolone acetonide disodium phosphate and triamcinolone acetonide dipotassium phosphate.

The above-mentioned corticosteroids normally are used in systemic treatment of anti-inflammatory diseases and disorders. Since it has been proved that by using a water-soluble form of a corticosteroid in long-circulating liposomes, having a specified small mean particle diameter, effective targeting of the drug to arthritic sites - by systemic administration - occurs, the present invention can advantageously be applied to corticosteroids, which - for a variety of reasons - normally are used for topical use. Such corticosteroids include for example alclomethasone dipropionate, amcinonide, beclomethasone monopropionate, betamethasone 17-valerate, ciclomethasone, clobetasol propionate, clobetasone butyrate, deprodone propionate, desonide, desoxymethasone, dexamethasone acetate, diflucortolone valerate, diflurasone diacetate, diflucortolone, difluprednate, flumetasone pivalate, flunisolide, fluocinolone acetonide acetate, fluocinonide, fluocortolone pivalate, fluormetholone acetate, fluprednidene acetate, halcinonide, halometasone, hydrocortisone acetate, medrysone, methylprednisolone acetate, mometasone furoate, parametasone acetate, prednicarbate, prednisolone acetate, prednylidene, rimexolone, tixocortol pivalate and triamcinolone hexacetonide. Topical corticosteroids of special interest are e.g. budesonide, flunisolide and fluticasone propionate, which undergo fast, efficient clearance as soon as these drugs become available in the general circulation. By preparing a water soluble form of these steroids and encapsulating this into long-circulating liposomes in accordance with the present invention it is now possible to systemically administer such corticosteroids in order to reach site-specific drug delivery, thereby avoiding adverse effects associated with systemic treatment and overcoming problems, which are inherent to the corticosteroid, such as a fast clearance. In this respect budesonide disodium phosphate has appeared to be a salt of great interest.

The lipid components used in forming the liposomes may be selected from a variety of vesicle-forming lipids, such as phospholipids, sphingolipids and sterols. "Phospholipid" refers to any one phospholipid or combination of phospholipids capable of forming liposomes. Phosphatidylcholines (PC), including those obtained from natural sources or those that are partially or wholly synthetic, or of variable lipid chain length and unsaturation are suitable for use in the present invention. Preferred phospholipids contain saturated alkyl chains, such as DSPC, HSPC and DPPC, yielding a bilayer with a relatively high transition temperature. Cholesterol is preferred as a bilayer component and can form up to 50 mole% of the bilayer constituents.

Substitution (complete or partial) of these basic components by e.g. sphingomyelines and ergosterol appeared to be possible. For effective encapsulation of the water-soluble corticosteroids in the liposomes, thereby avoiding leakage of the drug from the liposomes,

The liposomes in accordance with the present invention may be prepared according to methods used in the preparation of conventional liposomes. Passive loading of the active ingredients into the liposomes by dissolving the corticosteroid in the aqueous phase can result in sufficient amounts of encapsulated drug. However, active or remote loading is preferred, as with this method higher encapsulation efficiencies can be realized. With remote loading the temperature-sensitive corticosteroid esters may avoid the time-consuming and possible harmful extrusion step. Remote loading of corticosteroids can be realized using a pH gradient and involves the encapsulation of calcium acetate as a complexing agent in the liposomal interior.

Advantages of liposomes according to the invention over PEG-liposomes are: a higher encapsulation efficiency and a better drug to lipid ratio when corticosteroids are encapsulated. More importantly, less acute complement-related side effects may be expected with liposomes according to the invention when the liposomal formulation is injected intravenously.

The beneficial effects observed after a single injection of the water-soluble corticosteroid containing long-circulating liposomes according to the invention are very favourable when compared with the results obtained after repeated injections of the non-encapsulated water-soluble corticosteroid in different concentrations. The liposomes in accordance with the invention have shown an improved pharmacokinetic profile as compared with PEG-liposomes. Besides an increase of the AUC under the dexamethasone phosphate plasma concentration-time curve, also less free dexamethasone is observed in the circulation during the first hours after injection of liposomal dexamethasone phosphate.

The compositions according to the present invention can be advantageously used for the preparation of a medicament in the treatment of inflammatory diseases such as rheumatoid arthritis, osteoarthritis, multiple sclerosis, psoriasis, inflammatory bowel syndrome, colitis, Crohn's disease in human being suffering from the said diseases. Application in oncology is also useful.

The following examples further illustrate the invention.

### EXAMPLES

### Reference example

### Preparation of dexamethasone phosphate-containing PEG-liposomes

694 mg of dipalmitoyl phosphatidylcholine (DPPC) (Lipoid Ludwigshafen), 193 mg of cholesterol (Sigma Aldrich) and 206 mg of PEG-distearoylphosphatidylethanol-amine (PEG-DSPE) (Avanti Polar Lipids) were weighed and mixed in a 100 ml round-bottom flask. The lipids were dissolved in about 30 ml of ethanol. Thereafter evaporating to dryness in a Rotavapor during 1 hour under vacuum at 40°C, followed by flushing with nitrogen gas during 1 hour took place.

1000 mg of dexamethasone disodium phosphate (OPG Nieuwegein) were weighed and dissolved in 10 ml of sterilised water. The solution was added to the dry lipid film and shaked during five minutes in the presence of glass beads in order to enable complete hydration of the lipid film.

The liposomal suspension was transferred to an extruder (Avestin, maximum volume 15 ml) and extruded at room temperature under pressure, using nitrogen gas, 6 times through 2 pore filters one placed on top of the other, having a pore size of 200 and 100 nm respectively, 100 and 50 nm respectively and 50 and 50 nm respectively. Subsequently the liposomal suspension was dialysed in a dialysing compartment (Slide-A-Lyzer, 10.000 MWCO) 2 times during 24 hours against 1 liter of sterilised PBS.

The mean particle size of the liposomes was determined by means of light scattering (Malvern Zeta-sizer) and was found to be 93.1 ± 1.2 nm, the polydispersity index being 0.095 ± 0.024. The encapsulation efficiency of the dexamethasone phosphate was determined by means of a HPLC method and was found to be 4.8%. The phospholipid content was determined by lipid destruction using perchloric acid followed by phosphate determination and was 40.0 µmo1/ml. The drug to lipid ratio was found to be 0.12. The suspension of liposomes was stored in a nitrogen atmosphere at 4°C and found to be stable for about 2 months.

### Example 1

### Preparation of dexamethasone phosphate-containing liposomes

750 mg of dipalmitoyl phosphatidylcholine (DPPC) (Lipoid Ludwigshafen) and 193 mg of cholesterol (Sigma Aldrich) were weighed into and mixed in a 100 ml round-bottom flask. The lipids were dissolved in about 30 ml of ethanol. Thereafter evaporating to dryness in a Rotavapor during 1 hour under vacuum at 40°C, followed by flushing with nitrogen gas during 1 hour took place.

1000 mg of dexamethasone disodium phosphate (OPG Nieuwegein) were weighed and dissolved in 10 ml of sterilised water. The solution was added to the dry lipid film and shaken during five minutes in the presence of glass beads in order to enable complete hydration of the lipid film.

The liposomal suspension was transferred to an extruder (Avestin, maximum volume 15 ml) and extruded at room temperature as described in the reference example.

The mean particle size of the liposomes was determined as described in the reference example and was found to be 102.0 ± 4.3 nm, the polydispersity index being 0.12 ± 0.05. The encapsulation efficiency of dexamethasone phosphate was 8.4 %. The phospholipid concentration was 26.6 umol/ml. The drug to lipid ratio was found to be 0.32. The suspension of liposomes was stored in a nitrogen atmosphere at 4°C.

### Example 2

### Preparation of dexamethasone phosphate containing liposomes

Example 1 was repeated but instead of DPPC, distearoyl phosphatidylcholine (DSPC) was used as the main lipid component. Hydration was performed as described in the previous examples, however the suspension was repeatedly heated during the hydration process and took 15 minutes instead of 5 minutes as described above. After hydration the liposome dispersion was extruded as described in example 1, however the extrusion process was performed at 65 °C. The mean particle size of the liposomes was determined as described in the reference example and was found to be 102.9 ± 0.5 nm, the polydispersity index being 0.26 ± 0.015. The encapsulation efficiency of dexamethasone phosphate was 17.5 %. The phospholipid concentration was 57.5 umol/ml. The drug to lipid ratio was found to be 0.30. The suspension of liposomes was stored in a nitrogen atmosphere at 4°C.

### Example 3

### Preparation of dexamethasone phosphate containing liposomes

Example 2 was repeated. Instead of 100 mg/ml dexamethasone phosphate, 10 ml of a 50 mg/ml dexamethasone phosphate solution was used for hydration of the lipid film. After hydration the liposome dispersion was extruded as described in example 2 at 65 °C. After extrusion through two filters with a pore size of 50 nm, the liposome dispersion was extruded 6 times through two filters having a pore size of 50 and 30 rim respectively and two filters, both having a pore size of 30 mn. The mean particle size of the liposomes was determined as described in the reference example and was found to be 63.1 ± 0.7 nm, the polydispersity index being 0.20 ± 0.021. The encapsulation efficiency of dexamethasone phosphate was 14.4 %. The phospholipid concentration was 63.2 µmol/ml. The drug to lipid ratio was found to be 0.11. The suspension of liposomes was stored in a nitrogen atmosphere at 4°C.

### Example 4

### Preparation of dexamethasone phosphate containing liposomes

Example 2 was repeated. Instead of 750 mg, 694 mg DSPC was used. In addition, 112 mg negatively charged dipalmitoyl phosphatidyl glycerol was added as a lipid bilayer component. Hydration and extrusion was performed as described in example 2. The mean particle size of the liposomes was 95.1 ± 0.9 nm, the polydispersity index being 0.12 ± 0.018. The encapsulation efficiency of dexamethasone phosphate was 3.0 %. The phospholipid concentration was 39.0 µmol/ml. The drug to lipid ratio was found to be 0.08. The suspension of liposomes was stored in a nitrogen atmosphere at 4°C.

### Example 5

### Preparation of dexamethasone phosphate containing liposomes

Example 3 was repeated. Instead of 750 mg, 694 mg DSPC was used. In addition, 112 mg negatively charged dipalmitoyl phosphatidyl glycerol was added as a lipid bilayer component. Hydration and extrusion was performed as described in example 3. The mean particle size of the liposomes was 65.3 ± 0.5 nm, the polydispersity index being 0.17 ± 0.021. The encapsulation efficiency of dexamethasone phosphate was 3.0 %. The phospholipid concentration was 53.8 amol/ml. The drug to lipid ratio was found to be 0.06. The suspension of liposomes was stored in a nitrogen atmosphere at 4°C.

### Example 6

### Comparative kinetics of liposomal dexamethasone phosphate and free dexamethasone in the circulation after a single intravenous injection to the rat

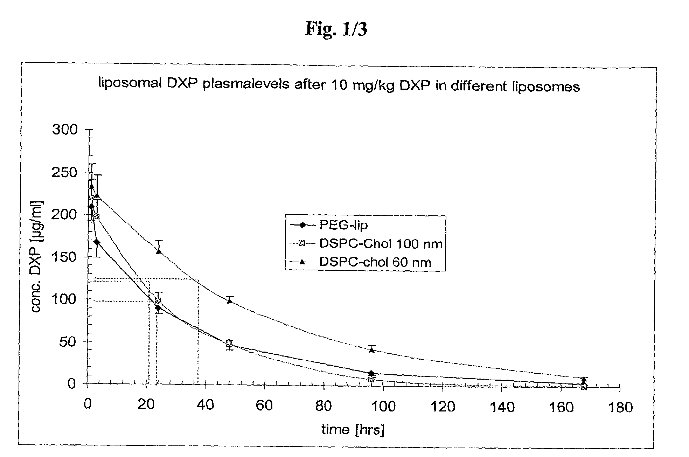

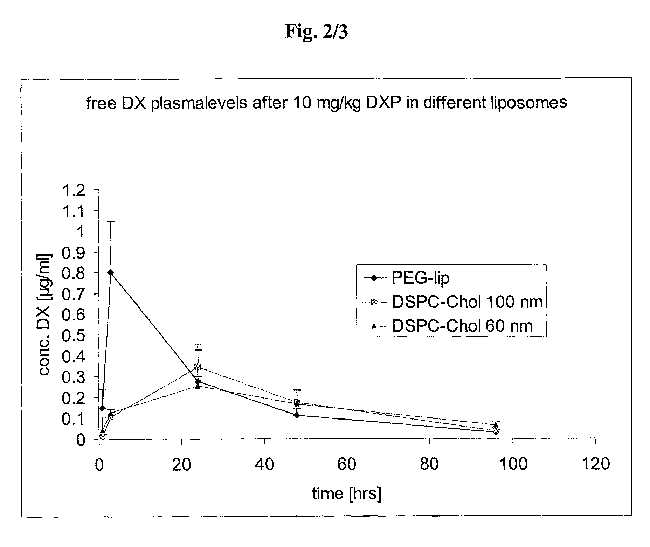

Male rats (Lewis (outbred, SPF-Quality) (Maastricht University, The Netherlands)) had free access to standard pelleted laboratory animal diet (Altromin, code VRF 1, Lage, Germany) and to tap-water. Single-dose intravenous injection of liposomal preparations, each containing 10 mg/kg dexamethasone phosphate was given into the tail-vein. Blood samples were collected from the tail vein of each rat at the following time points post-dose: 1, 4, 24 and 48 hours, 4 days and 1 week. The amount of sample collected was approx. 500 µlper sampling event.
Sampled blood was transferred into EDTA-containing tubes, centrifuged and the plasma fraction was stored at -80 °C. Extraction of both dexamethasone phosphate and dexamethasone from 200 µl plasma samples was performed with 2 ml ethyl acetate after adding phosphoric acid to lower the pH of the plasma fraction. The ethyl acetate fraction was evaporated under nitrogen and the extract was reconstituted in 150 µl of a mixture of ethanol/water 50/50. These solutions were transferred to a reversed phase HPLC system equipped with C18 column using an acetonitrile/water mixture 25/75 with pH=2 as the mobile phase. Detection was performed with an UV-detector at 254 nm.
The results are shown in Figure 1 and 2.

**Tabel 1:**

| liposomes: properties | | | | | |
|---|---|---|---|---|---|
| | Lipid content | lipid loss | DXP content | Encaps. Effic. | Steroid to lipid ratio |
| DPPC:PEGPE:Chol 90nm | 39.975 mg/ml | 46.7 % | 4.75 mg/ml | 4.75 % | 0.12 |
| DSPC:Chol 100nm | 57.45 mg/ml | 23.4 % | 17.5 mg/ml | 17.5 % | 0.30 |
| DSPC:Chol 60nm | 63.225 mg/ml | 15.7 % | 7.2 mg/ml | 14.4 * % | 0.11 |
| DSPC:Chol:DPPG 90nm | 39.0375 mg/ml | 48.0 % | 3 mg/ml | 3 % | 0.08 |
| DSPC:Chol:DPPG 60nm | 53.775 mg/ml | 28.3 % | 3 mg/ml | 3 % | 0.06 |
| DPPC:Chol 100nm | 26.625 mg/ml | 64.5 % | 8.4 mg/ml | 8.4 % | 0.32 |

### Example 7

### Assessment of therapeutic efficacy

Lewis rats were immunized subcutaneously at the tail base with heat-inactivated *Mycobacterium tuberculosis* in incomplete Freund's adjuvant. Paw inflammation started between 9 and 12 days after immunization, reached maximum severity approximately after 20 days, and then gradually resolved.

Assessment of the disease was performed by visually scoring paw inflammation severity, maximum score 4 per paw, and measuring disease-induced body weight loss. The therapeutic efficacy of 10 mg/kg liposomal dexamethasone phosphate, prepared according to reference example, example 2 and 3 were evaluated against PBS as the control treatment. Rats were treated when the average score > 6 (at day 14 or 15 after disease induction).

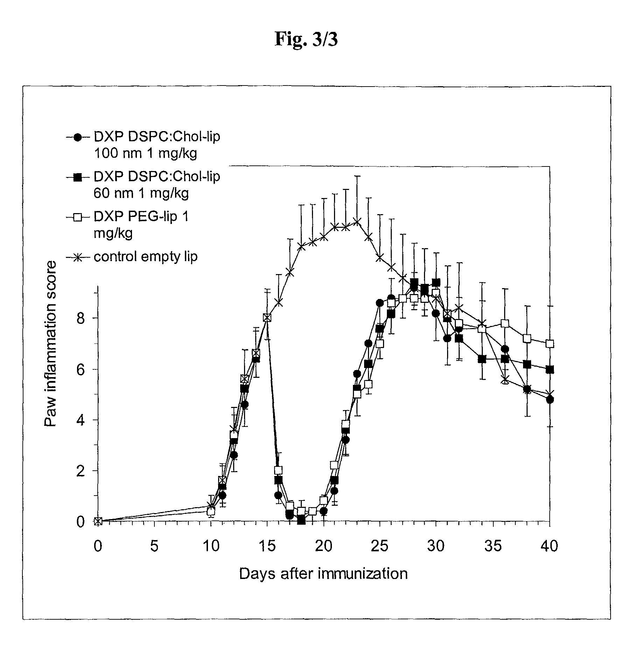

A complete remission of the inflammation process was observed within 3 days after treatment with a single dose of 10 mg/kg liposomal dexamethasone phosphate. All three preparations induced the same therapeutic effect in the disease model (results shown in Figure 3).

## Claims

1. A pharmaceutical composition for parenteral administration, the composition comprising liposomes composed of non-charged vesicle-forming lipids, optionally including not more than 5 mol% of charged vesicle-forming lipids, which liposomes have a selected mean particle diameter in the size range between about 40 - 200 nm and contain a corticosteroid for the site-specific treatment of inflammatory disorders, **characterised in that** the corticosteroid is present in a water soluble form.

2. Composition according to claim 1, **characterised in that** the corticosteroid is selected from the group consisting of systemically administered corticosteroids.

3. Composition according to claim 2, **characterised in that** the systemically administered corticosteroids are selected from the group consisting of prednisolone, dexamethasone and methylprednisolone.

4. Composition according to claim 1, **characterised in that** the corticosteroid is selected from the group of topically applied corticosteroids.

5. Composition according to claim 4, **characterised in that** the topically applied corticosteroids are selected from the group consisting of budesonide, flunisolide and fluticasone propionate.
